# EUROPEAN PATENT SPECIFICATION

(11) **EP 3 847 150 B1**
(45) Date of publication and mention of the grant of the patent: **22.11.2023**
(21) Application number: 19772911.4
(22) Date of filing: 04.09.2019
(51) Int. Cl.: C07C 37/20, C07C 37/74, C07C 37/82, C07C 39/16

(54) **PROCESS FOR PRODUCING BISPHENOL-A**
VERFAHREN ZUR HERSTELLUNG VON BISPHENOL-A
PROCÉDÉ DE PRODUCTION DE BISPHÉNOL-A

(30) Priority: 05.09.2018 US 201862727230 P
(43) Date of publication of application: 14.07.2021
(73) Proprietor: Badger Licensing, LLC, Boston, MA 02111 (US)
(72) Inventor: PALMER, David, Katy, Texas 77494 (US)
(74) Representative: McWilliams, David John
(86) International application number: PCT/US2019/049462
(87) International publication number: WO 2020/051186

(56) References cited:
- EP-A1- 1 985 602
- JP-A- 2000 229 899
- US-A- 4 766 254
- US-B1- 6 653 513

## Description

### FIELD

This invention relates to a process for producing bisphenol-A.

### BACKGROUND

Bisphenol-A (BPA), also referred to as 2,2-bis (4-hydroxyphenyl) propane or para, para-diphenylolpropane (p,p-BPA), is a commercially significant compound used to manufacture polycarbonates, other engineering thermoplastics and epoxy resins. BPA is produced commercially by the condensation of acetone and phenol and, in fact, BPA production is the largest consumer of phenol. The reaction may take place in the presence of a strong homogenous acid, such as hydrochloric acid, sulfuric acid, or toluene sulfonic acid, or in the presence of a heterogeneous acid catalyst, such as a sulfonated ion exchange resin. In recent years, acidic ion exchange resins have become the overwhelming choice as catalysts for the condensation reaction of bisphenol manufacture, and strongly acidic sulfonated polystyrene ion exchange resins are particularly useful in this regard.

Two different techniques for employing heterogeneous acid catalysts in bisphenols production predominate in industrial practice. In one technique, a co-catalyst promoter is freely circulated in the reactor with the reaction feed. It is used to enhance the selectivity and/or activity of the reaction. An alkanethiol, such as methyl or ethyl mercaptan, or a mercaptocarboxylic acid, such as 3-mercaptopropionic acid, is typically used as the freely circulating co-catalyst promoter in this technique. The acidic sites of the resin are left available, that is, largely unbound to the promoter. This provides flexibility in adjusting the optimal concentration of promoter given the particular reaction conditions in question.

In the second technique for employing heterogeneous acid catalysts in the production of bisphenols, the catalyst is modified by appending co-catalytic agents, such as thiazolidines and aminothiols, to some of the acid sites on the catalyst. For example, mercapto-promoter groups may be attached to backbone sulfonate ions of a cation exchange resin by covalent or ionic nitrogen linkages. The fixed promoter technique requires less direct handling and treatment of promoters than the freely circulating promoter process, but the ability to refine the process by differential treatment of the resin and promoter is greatly reduced.

Among the requirements for BPA, especially when used in the production of polycarbonates, are thermal stability and high purity due to stringent requirements for optical clarity and color in the finished application. It is therefore known from, for example, JP2000-229899 A to distill the BPA reaction effluent to remove acetone and alkylmercaptan and then contact the BPA-containing stream with an acidic ion exchange resin to decompose organosulfur impurities and generate alkylmercaptan which is subsequently removed. It is also known from US Patent No. 5,124,490 to contact BPA process streams with a basic anion exchange bed to improve the color and thermal stability of the BPA product by removal of trace amounts of leached acid from the strong acid, sulfonated ion exchange resin catalyst used in the acetone/phenol condensation reaction.

While treatment with a basic anion exchange resin is effective in purifying BPA process streams, if the process streams also contain residual mercaptan promoter, such as methyl or ethyl mercaptan, it is found that contact with the basic anion exchange resin can convert the mercaptan into dimethyl sulfide, dimethyl disulfide, diethyl disulfide, hydrogen sulfide or other sulfur species. This results in an uneconomic loss of the mercaptan promoter as well as requiring additional process steps to remove unwanted sulfur species.

There is therefore a need for an improved method of purifying BPA process streams produced using heterogeneous acid catalysts promoted with organic sulfur-containing compounds.

### SUMMARY

According to the present invention, it has now been found that to realize the quality benefits from contact with the anion exchange bed without loss of the mercaptan promoter, the BPA reaction effluent should first be distilled to remove the mercaptan promoter and unreacted acetone before contact with the anion exchange bed. Further, to maximize capture of trace acidity, contact with the anion exchange bed should preferably occur before the distilled stream undergoes crystallization or other methods to recover the BPA.

Thus, in one aspect, the invention resides in a process for producing bisphenol-A, the process comprising:
(a) reacting acetone and phenol in the presence of a catalyst system comprising an acidic heterogeneous catalyst and a catalyst promoter comprising at least one organic sulfur-containing compound to produce a reaction effluent comprising bisphenol-A, water, unreacted acetone, unreacted phenol and at least part of the catalyst promoter;
(b) distilling at least part of the reaction effluent to remove water, catalyst promoter and unreacted acetone, and leave a residual stream containing bisphenol A;
(c) contacting at least part of the residual stream with a basic anion exchange resin to produce a purified stream; and
(d) recovering bisphenol-A from the purified stream.

### BRIEF DESCRIPTION OF THE DRAWINGS

Figure 1 is a simplified flow diagram of a process for producing bisphenol-A according to one embodiment of the invention.
Figure 2 is a graph showing the variation with time on stream of the diethyl disulfide (DEDS) concentration in the product obtained in Example 1 using 10 g (dry basis) of the Lewatit@ MP 62 WS anion exchange resin at temperatures of 90°C, 110°C and 120°C.
Figure 3 is a graph showing the variation with time on stream of the diethyl disulfide (DEDS) concentration in the product obtained in Example 1 using different amounts of Lewatit@ MP 62 WS anion exchange resin at a temperature of 90°C.
Figure 4 is a graph showing the variation with time on stream of the diethyl disulfide (DEDS) concentration in the product obtained in Example 1 using of 10 g (dry basis) of the Lewatit@ MP 62 WS anion exchange resin and 10 g (dry basis) of the Amberlyst^{™} A26 anion exchange resin, both at a temperature of 90°C.

### DETAILED DESCRIPTION OF THE EMBODIMENTS

The present invention relates to the production of bisphenol-A (BPA) by condensation of acetone with phenol in the presence of a catalyst system comprising an acidic heterogeneous catalyst and a catalyst promoter comprising at least one organic sulfur-containing compound. The condensation reaction effluent comprises crude BPA product, typically containing acid impurities leached from the catalyst, water, at least part of the catalyst promoter and typically unreacted acetone and phenol. In particular, the present invention provides a simple and effective process for purifying the BPA product, thereby improving its color and thermal stability, while minimizing loss of catalyst promoter present in the reaction effluent stream.

Any known reaction system can be used to effect the condensation of acetone and phenol to produce BPA in accordance with the invention. Generally, the acetone and phenol are reacted in a reaction zone, whether in batch or continuous mode, at a temperature ranging from about 20°C to about 130°C, preferably from about 50°C to about 90°C.

The pressure conditions are not particularly limited and the reaction may proceed at atmospheric, sub atmospheric or super atmospheric pressure. However, it is preferred to run the reaction either without any externally induced pressure, or at sufficient pressure to force the reaction mixture across a catalyst bed or to force the reaction mixture upstream in a vertical reactor, or to maintain the contents of the reaction vessel in a liquid state if the reaction is run at a temperature above the normal boiling point of any ingredient. The pressure and temperature should be set under conditions to retain the reactants in the liquid phase in the reaction zone. The temperature may exceed 130°C, but should not be so high as to degrade any of the ingredients in the reaction vessel, nor should it be so high as to degrade the reaction product or promote the synthesis of a substantial amount of unwanted by-products.

The reactants are introduced into the reaction zone under conditions to assure a molar excess of the phenol over the acetone. For example, the molar ratio of the phenol to the acetone is preferably at least about 2:1, more preferably at least about 4:1, and up to about 25:1.

The condensation reaction is conducted in the presence of an acidic heterogeneous catalyst promoted by at least one organic sulfur-containing compound. Suitable catalysts include molecular sieves, salts of heteropolyacids partially neutralized and insolubilized, and acidic cation exchange resins. Preferred condensation catalysts are cation exchange resins and especially those having a cation exchange capacity of at least about 0.5 and, more preferably, greater than about 4.0, meq/g dry weight. Particularly preferred are sulfonated cation exchange resins, such as sulfonated styrene-divinylbenzene copolymers, sulfonated cross-linked styrene polymers, phenol-formaldehyde-sulfonic acid resins, benzene-formaldehyde-sulfonic acid resins, perfluorinated sulfonic acid resins and the like. These include resins sold under such trade names as Amberlites or Amberlysts (Rohm and Haas Co.), DOWEX (Dow Chemical Co.), Permutit QH (Permutit Co.), Chempro (Chemical Process Co.), catalysts from Purolite, Lewatit^{®} (LANXESS Deutschland GmbH), NAFION^{®} (DuPont) and the like. Strong acid sulfonated styrene-divinylbenzene copolymer resins are preferred. Suitable cation exchange resins are made from sulfonated polymerized styrene monomer which has been cross linked with from about 1% to about 8% divinylbenzene (resin). Specific examples of suitable sulfonated resins are Amberlyst^{®} 131, Lewatit@ K-1221, Purolite^{®} CT-122, Purolite^{®} CT-124, Diaion^{™} SK104H, Tulsion^{®} 38, and Dowex^{®} 50WX4.

The condensation catalyst system also includes at least one organic sulfur-containing promoter, which generally contains at least one thiol, S-H, group. Such thiol promoters can be either ionically or covalently bonded to the heterogeneous acid catalyst or unbound to the heterogeneous acid catalyst and added separately to the condensation reaction. Non-limiting examples of bound promoters include mercaptoalkylpyridines, mercaptoalkylamines, thiazolidines and aminothiols. Non-limiting examples of unbound promoters include alkyl mercaptans, such as methyl mercaptan (MeSH) and ethyl mercaptan, mercaptocarboxylic acids, such as mercaptopropionic acid, and mercaptosulfonic acids.

The amount of organic sulfur-containing promoter employed in the catalyst system depends on the particular acidic heterogeneous catalyst employed. In general, however, the organic sulfur-containing promoter is employed in an amount from 2 to 30 mol %, such as 5 to 20 mol %, based on the acid group (sulfonic group) in the acid ion exchanger.

Where the unbound thiol promoter is methyl mercaptan, and the carbonyl compound is acetone, 2,2-bis(methylthio) propane (BMTP) is formed in the presence of an acidic catalyst. In the presence of a hydrolyzing agent, BMTP dissociates in the reaction zone into methyl mercaptan and acetone as acetone is condensed with phenol to form BPA. A convenient hydrolyzing agent is water, which may be introduced into any of the feed charges, directly into the reaction zone, or may be produced in situ by the condensation reaction between the carbonyl compound and the phenolic compound. A molar ratio of water to BMTP catalyst promoter ranging from about 1:1 to about 5:1 is sufficient to adequately hydrolyze the BMTP catalyst promoter. This quantity of water is produced in situ under typical reaction conditions. Thus, additional water does not need to be introduced into the reaction zone, although water may optionally be added if desired.

Any suitable reactor may be used as the reaction zone. The reaction can occur in a single reactor, or in a plurality of reactors connected in series or in parallel. The reactor can be a back mixed or plug flow reactor, and the reaction can be conducted in a continuous or batch mode, and the reactor can be oriented to produce an up-flow or down-flow stream. In the case of a fixed bed flow system, the liquid space velocity of the mixture of the raw materials supplied to the reactor is usually 0.2 to 50 hr⁻¹. In the case of a suspended bed batch system, the amount of the strongly acid ion exchange resin used, although variable depending on the reaction temperature and pressure, is usually 20 to 100% by weight based on the mixture of the raw materials. The reaction time is usually 0.5 to 5 hours.

The main products of the condensation reaction are the desired bisphenol isomer, bisphenol-A, and water together with various by-products, including other diphenylolpropane isomers, such as 2-(4-hydroxyphenyl)-2-(2-hydroxyphenyl) propane or o,p-BPA, trisphenols and other impurities, such as chromans and indanes. In addition, the condensation reaction effluent also contains unreacted phenol and unreacted acetone, as well as at least part of the organic sulfur-containing promoter.

The crude condensation reaction effluent also contains trace amounts of polysulfonic acid groups which are leached from the sulfonated ion exchange catalyst during the condensation reaction. These polysulfonic acid groups can cause degradation of the BPA product melt, increase the melt color, and decrease the thermal stability of the BPA product, particularly in high clarity polycarbonate applications. These effects can be ameliorated by contacting the crude reaction effluent with a basic anion exchange resin. However, it has been found that, although treatment with a basic anion exchange resin is effective in removing acid groups from BPA process streams, if the process streams also contain residual mercaptan promoter, the treatment also converts the mercaptan into dimethyl sulfide, dimethyl disulfide, diethyl disulfide, hydrogen sulfide or other sulfur species. This results in an uneconomic loss of the mercaptan promoter as well as requiring additional process steps to remove any sulfur species.

To obviate this problem, in the present process the crude condensation reaction effluent, before any contact with a basic anion exchange resin, is distilled to remove at least part of the catalyst promoter, unreacted acetone, unreacted phenol and water from the crude effluent and leave a residual stream containing phenol, BPA, other diphenylolpropane isomers, trisphenols and any other heavy by-products. Conveniently, the distillation of the crude condensation reaction effluent is conducted at a temperature from 155 to 185°C and a pressure from 50 to 85 kPa-a.

At least part of the catalyst promoter, unreacted acetone and unreacted phenol removed by the distillation step is preferably recycled to the condensation reaction.

The residual stream remaining after the distillation step typically contains less than 1,000 ppm by weight of water, such as less than 100 ppm by weight of water, less than 500 ppm by weight acetone, such as less than 50 ppm by weight of acetone, and less than 100 ppm by weight sulfur-containing catalyst promoter, such as less than 10 ppm by weight of sulfur-containing catalyst promoter. The residual stream is then contacted with a basic anion exchange resin to reduce the level of acid groups leached from the strong acid, sulfonated ion exchange condensation catalyst and produce a purified stream. Contacting with the basic anion exchange resin is conducted at a temperature from 80 to 120°C, such as by downward flow of the residual stream through a bed of basic anion exchange resin. A suitable anion exchange resin can be a strong or weak base anionic exchange resin, preferably a weak-base anionic exchange resin containing primary, secondary or tertiary amine groups. Examples of commercially available basic anion exchange resins are Lewatit@ MP 62, Amberlyst^{™} A21, Amberlyst^{™} A26, and Diaion^{™} WA-21.

Any method known to those of skill in the art may be employed to recover the desired BPA product from the resultant purified stream. One preferred method of BPA recovery is crystallization. The crystallization process can be one of several known techniques for BPA purification, preferably crystallization of an equimolar adduct with phenol. The formed crystals are then separated from the mother liquor by filtration or centrifugation. The separated mother liquor is then recycled to the condensation reactor. In one embodiment, the mother liquor is separately contacted with a strong acid, sulfonated ion exchange reaction catalyst to convert impurities, especially other isomers of diphenylolpropane, to BPA before the reaction product is returned to the condensation reactor.

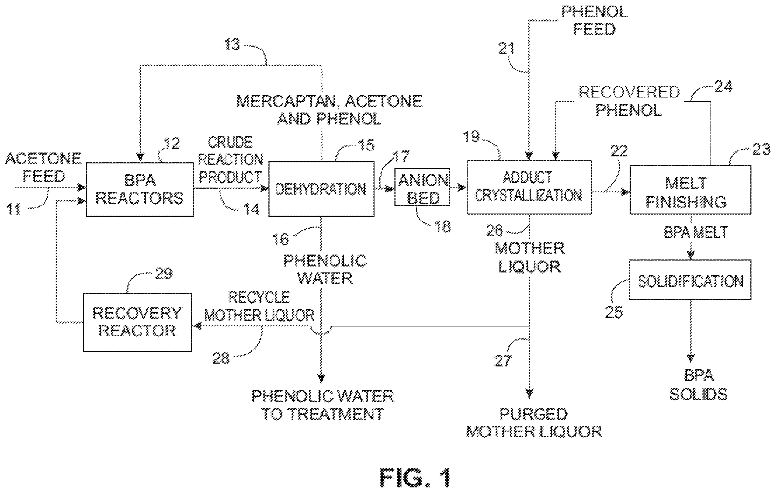

One embodiment of the present process is shown in Figure 1, in which fresh acetone is fed by line 11 to a condensation reactor 12, which also receives recycled acetone, phenol and organic sulfur-containing promoter via line 13. The condensation reactor 12 contains an acidic heterogeneous catalyst, preferably a sulfonated ion exchange resin, and is operated under conditions such that the acetone and phenol react to produce condensation reaction products comprising BPA and water together with various by-products, including other isomers of diphenylolpropane, trisphenols and heavy impurities, such as chromans and indanes.

The effluent from the condensation reactor 12, containing the condensation reaction products as well as unreacted acetone and phenol and at least part of the catalyst promoter, is fed via line 14 to one or more distillation columns 15 which are operated to remove (i) a first light stream composed of most of the unreacted acetone and the catalyst promoter, (ii) a second intermediate stream composed of most of the water and some unreacted phenol in the effluent, and (iii) a third stream composed primarily of unreacted phenol. The first stream (i) and the third stream (iii) are collected and recycled to the condensation reactor 12 via line 13, while the second light stream (ii) is collected and fed to waste water treatment (not shown) via line 16.

After removal of the first light stream (i), the second intermediate stream (ii), and the third stream (iii), the remaining residual stream, which contains most of the bisphenol component of the condensation reactor effluent and the remaining unreacted phenol, is fed by line 17 to a bed 18 of a basic anion exchange resin for removal of leached polysulfonic acid groups. After leaving the bed 18, the purified residual stream is fed to a crystallization unit 19 which receives fresh phenol through line 21. An adduct of phenol with BPA is selectively crystallized in the crystallization unit 19, separated from the mother liquor by filtration or centrifugation and fed by line 22 to a melt finishing unit 23. The adduct crystals are melted in the finishing unit 23 to separate the phenol from the BPA, with the phenol being recycled to the crystallization unit 19 via line 24 and the BPA being fed to a solidification device 25.

The mother liquor remaining after recovery of the BPA/phenol adduct is removed from the crystallization unit 19 via line 26 and divided into a purge stream 27 and a recycle stream 28, which is returned to the condensation reactor 12. In some embodiments, prior to return to the condensation reactor 12, the mother liquor recycle stream is separately contacted with a strong acid, sulfonated ion exchange reaction catalyst 29 to convert impurities, especially other isomers of diphenylolpropane, to the preferred BPA isomer.

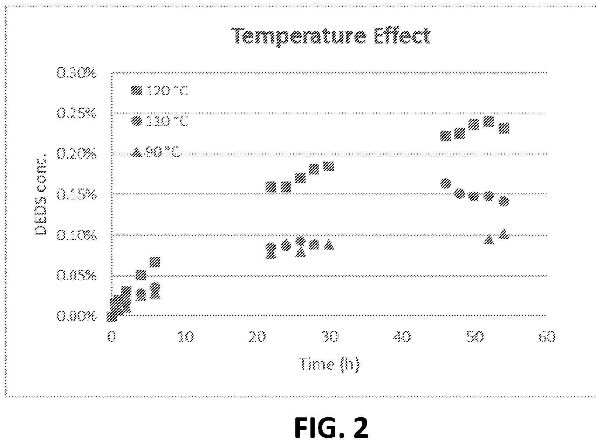

The invention will now be more particularly described with reference to the following non-limiting Examples and Figures 2 to 4 of the accompanying drawings.

### Example 1

A synthetic mixture composed of 70 wt.% phenol, 25 wt.% BPA, 2 wt.% water, 2 wt.% ethyl mercaptan and 1 wt.% acetone was prepared to simulate the effluent obtained in the condensation reaction of phenol and acetone in the presence of ethyl mercaptan promoter, a representative alkyl mercaptan promoter.

A series of tests were performed in which 200 g of this synthetic reaction effluent mixture was contacted in a closed autoclave with varying amounts of the basic anion exchange resin Lewatit^{®} MP 62 WS at temperatures of 90°C, 110°C and 120°C, and with 10 g of the basic anion exchange resin Amberlyst^{™} A26 at 90°C. In each case, the anion exchange resin was washed with 10 bed volumes of demineralized water before the tests.

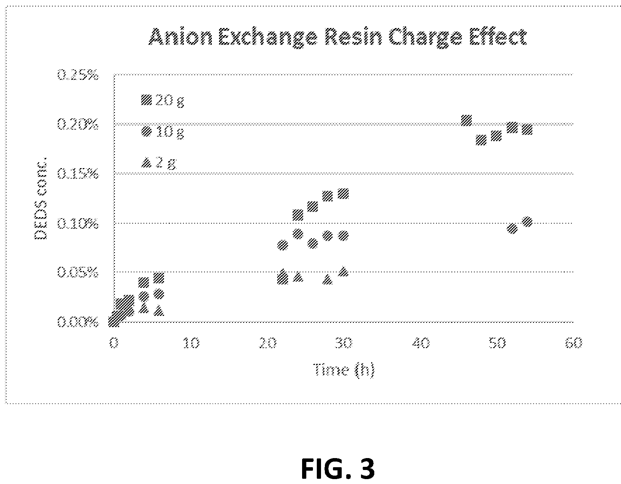

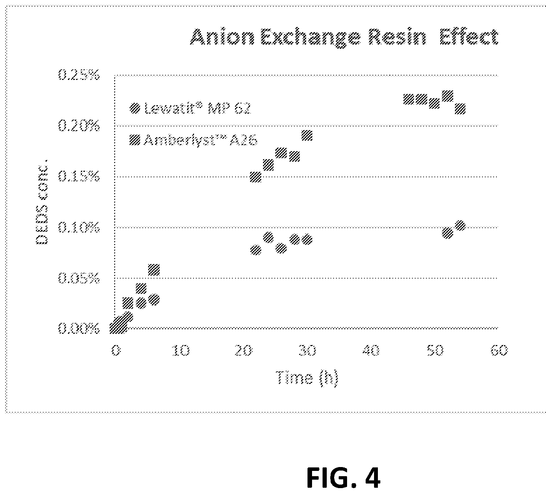

The contents of the autoclave were sampled at various times throughout each test, and the samples were analyzed with GC-FID/SCD to measure the identity and amount of any sulfide species generated. The results are summarized in Figures 2 to 4, and illustrate that diethyl disulfide (DEDS) was created during contact with the basic anion exchange resin in the presence of ethyl mercaptan promoter. Specifically, the results show that the DEDS concentration in the reaction product increased with reaction temperature (Figure 2) and anion exchange resin load (Figure 3) using the Lewatit@ MP 62 WS anion exchange resin. Figure 4 shows that the Amberlyst^{™} A26 anion exchange resin produced more DEDS than the Lewatit@ MP 62 WS anion exchange resin at the same temperature, 90°C, possibly reflecting the stronger basic sites associated with the former.

### Example 2

A test similar to those of Example 1 was conducted, in which 200 g of a synthetic mixture composed of 70 wt.% phenol, 28 wt.% BPA, and 2 wt.% ethyl mercaptan was contacted with 10 g of washed Lewatit@ MP 62 WS anion exchange resin in an autoclave at 100°C. Over the 24 hours of the test it was found that the ethyl mercaptan content of the mixture decreased as an increasing amount of DEDS was detected. The test demonstrates the loss of mercaptan promoter to unwanted sulfur species if the anion exchange resin is not located downstream of a distillation step to remove the mercaptan from the condensation effluent.

### Example 3

A synthetic mixture composed of 70 wt.% phenol and 30 wt.% BPA was prepared to simulate the residual stream remaining after distillation of the reaction effluent of the condensation of phenol and acetone to remove water, catalyst promoter and unreacted acetone. 200 g of this synthetic residual mixture was contacted with 10 g of washed Lewatit@ MP 62 WS anion exchange resin in an autoclave at 100°C. No DEDS was detected after 24 hours on stream, demonstrating that DEDS was not created during contact with the anion exchange resin when the ethyl mercaptan promoter was not present in the mixture.

The same result (no DEDS detected) was obtained when the test was repeated with washed Amberlyst^{™} A26 anion exchange resin.

### Examples 4 to 6

The test of Example 3 was repeated with washed Lewatit@ MP 62 WS anion exchange resin, but with varying synthetic mixtures composed of 70 wt.% phenol, 28 wt.% BPA and 2 wt.% water (Example 4); 70 wt.% phenol, 29 wt.% BPA and 1 wt.% acetone (Example 5); and 70 wt.% phenol, 27 wt.% BPA, 2 wt.% water and 1 wt.% acetone (Example 6). In each case, no DEDS was detected after 24 hours on stream. These examples show that DEDS was not formed during contact with the anion exchange resin in the absence of the ethyl mercaptan promoter, and the presence of water and/or acetone in the mixture did not alter these results.

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

## Claims

1. A process for producing bisphenol-A, the process comprising:
(a) reacting acetone and phenol in the presence of a catalyst system comprising an acidic heterogeneous catalyst and a catalyst promoter comprising at least one organic sulfur-containing compound to produce a reaction effluent comprising bisphenol-A, water, unreacted acetone, unreacted phenol and at least part of the catalyst promoter;
(b) distilling at least part of the reaction effluent to remove water, catalyst promoter and unreacted acetone, and leave a residual stream containing bisphenol A;
(c) contacting at least part of the residual stream with a basic anion exchange resin to produce a purified stream; and
(d) recovering bisphenol-A from the purified stream.

2. The process of claim 1, wherein the acidic heterogeneous catalyst comprises a sulfonated ion exchange resin.

3. The process of claim 1 or claim 2, wherein the catalyst promoter comprises at least one organic sulfur-containing compound selected from the group consisting of alkyl mercaptans, mercaptocarboxylic acids, mercaptosulfonic acids, mercaptoalkylpyridines, mercaptoalkylamines, thiazolidines and aminothiols.

4. The process of any one of claims 1 to 3, wherein the distilling (b) is conducted at a temperature from 155 to 185°C and a pressure from 50 to 85 kPa-a.

5. The process of any one of claims 1 to 4, wherein the residual stream contains less than 1,000 ppm by weight of water, less than 500 ppm by weight acetone, and less than 100 ppm by weight sulfur-containing catalyst promoter.

6. The process of any one of claims 1 to 5 and further comprising:
(e) recycling at least part of the catalyst promoter removed in the distilling (b) to the reacting step (a).

7. The process of any one of claims 1 to 6, wherein the contacting (c) is conducted at a temperature from 80 to 120°C.

8. The process of any one of claims 1 to 7, wherein the contacting (c) is conducted by downward flow of the residual stream through a bed of basic anion exchange resin.

9. The process of any one of claims 1 to 8, wherein the anion exchange resin is a weak-base anionic exchange resin, containing primary, secondary or tertiary amine groups.

10. The process of any one of claims 1 to 9, wherein the recovering (d) comprises crystallization to separate the purified stream into a solid bisphenol-A-containing product and a mother liquor stream.

11. The process of claim 10, wherein the mother liquor stream comprises other isomers of diphenylolpropane than bisphenol-A and the process further comprises:
(f) contacting the mother liquor with a sulfonated acidic ion exchange catalyst under conditions to convert at least some of the other isomers of diphenylolpropane to bisphenol-A and produce an isomerized product stream.

12. The process of claim 11 and further comprising:
(g) supplying at least part of the isomerized product stream to the reacting step (a).

## Patentansprüche

1. Verfahren zur Herstellung von Bisphenol-A, das Folgendes umfasst:
(a) Umsetzen von Aceton und Phenol in Gegenwart eines Katalysatorsystems, das einen sauren heterogenen Katalysator und einen mindestens eine organische schwefelhaltige Verbindung umfassenden Katalysatorpromotor umfasst, zur Bildung eines Reaktionsaustragsstroms, der Bisphenol-A, Wasser, nicht umgesetztes Aceton, nicht umgesetztes Phenol und mindestens einen Teil des Katalysatorpromotors umfasst;
(b) Destillieren mindestens eines Teils des Reaktionsaustragsstroms zur Entfernung von Wasser, Katalysatorpromotor und nicht umgesetztem Aceton und Zurücklassung eines Bisphenol A enthaltenden Reststroms;
(c) Inkontaktbringen mindestens eines Teils des Reststroms mit einem basischen Anionenaustauscherharz zur Bildung eines gereinigten Stroms; und
(d) Gewinnen von Bisphenol-A aus dem gereinigten Strom.

2. Verfahren nach Anspruch 1, wobei der saure heterogene Katalysator ein sulfoniertes Ionenaustauscherharz umfasst.

3. Verfahren nach Anspruch 1 oder Anspruch 2, wobei der Katalysatorpromotor mindestens eine organische schwefelhaltige Verbindung aus der Gruppe bestehend aus Alkylmercaptanen, Mercaptocarbonsäuren, Mercaptosulfonsäuren, Mercaptoalkylpyridinen, Mercaptoalkylaminen, Thiazolidinen und Aminothiolen umfasst.

4. Verfahren nach einem der Ansprüche 1 bis 3, wobei das Destillieren (b) bei einer Temperatur von 155 bis 185 °C und einem Druck von 50 bis 85 kPa-a durchgeführt wird.

5. Verfahren nach einem der Ansprüche 1 bis 4, wobei der Reststrom weniger als 1000 Gew.-ppm Wasser, weniger als 500 Gew.-ppm Aceton und weniger als 100 Gew.-ppm schwefelhaltigen Katalysatorpromotor enthält.

6. Verfahren nach einem der Ansprüche 1 bis 5 und ferner umfassend:
(e) Rezyklieren mindestens eines Teils des bei dem Destillieren (b) entfernten Katalysatorpromotors zum Umsetzungsschritt (a) .

7. Verfahren nach einem der Ansprüche 1 bis 6, wobei das Inkontaktbringen (c) bei einer Temperatur von 80 bis 120 °C durchgeführt wird.

8. Verfahren nach einem der Ansprüche 1 bis 7, wobei das Inkontaktbringen (c) durch Abwärtsströmen des Reststroms durch eine Schüttung von basischem Anionenaustauscherharz durchgeführt wird.

9. Verfahren nach einem der Ansprüche 1 bis 8, wobei es sich bei dem Anionenaustauscherharz um ein schwach basisches Anionenaustauscherharz mit primären, sekundären oder tertiären Amingruppen handelt.

10. Verfahren nach einem der Ansprüche 1 bis 9, wobei das Gewinnen (d) Kristallisation zur Trennung des gereinigten Stroms in ein festes Bisphenol A enthaltendes Produkt und einen Mutterlaugestrom umfasst.

11. Verfahren nach Anspruch 10, wobei der Mutterlaugestrom andere Isomere von Diphenylolpropan als Bisphenol-A umfasst und das Verfahren ferner Folgendes umfasst:
(f) Inkontaktbringen der Mutterlauge mit einem sulfonierten sauren Ionenaustauschkatalysator unter Bedingungen zur Umwandlung mindestens einiger der anderen Isomere von Diphenylolpropan in Bisphenol-A und Bildung eines Stroms von isomerisierten Produkten.

12. Verfahren nach Anspruch 11 und ferner umfassend:
(g) Zuführen mindestens eines Teils des Stroms von isomerisierten Produkten zum Umsetzungsschritt (a) .

## Revendications

1. Procédé pour la production de bisphénol A, le procédé comprenant :
(a) la mise en réaction d'acétone et de phénol en la présence d'un système de catalyseur comprenant un catalyseur hétérogène acide et un promoteur de catalyseur comprenant au moins un composé contenant du soufre organique pour produire un effluent de réaction comprenant du bisphénol A, de l'eau, de l'acétone n'ayant pas réagi, du phénol n'ayant pas réagi et au moins une partie du promoteur de catalyseur ;
(b) la distillation d'au moins une partie de l'effluent de réaction pour éliminer de l'eau, du promoteur de catalyseur et de l'acétone n'ayant pas réagi, et laisser un flux résiduel contenant du bisphénol A ;
(c) la mise en contact d'au moins une partie du flux résiduel avec une résine d'échange d'anions basique pour produire un flux purifié ; et
(d) la récupération de bisphénol A du flux purifié.

2. Procédé selon la revendication 1, le catalyseur hétérogène acide comprenant une résine échangeuse d'ions sulfonatée.

3. Procédé selon la revendication 1 ou la revendication 2, le promoteur de catalyseur comprenant au moins un composé contenant du soufre organique choisi dans le groupe constitué par des alkyl-mercaptans, des acides mercaptocarboxyliques, des acides mercaptosulfoniques, des mercaptoalkylpyridines, des mercaptoalkylamines, des thiazolidine et des aminothiols.

4. Procédé selon l'une quelconque des revendications 1 à 3, la distillation (b) étant conduite à une température de 155 à 185 °C et à une pression de 50 à 85 kPa-a.

5. Procédé selon l'une quelconque des revendications 1 à 4, le flux résiduel contenant moins de 1 000 ppm en poids d'eau, moins de 500 ppm en poids d'acétone et moins de 100 ppm en poids de promoteur de catalyseur contenant du soufre.

6. Procédé selon l'une quelconque des revendications 1 à 5 et comprenant en outre :
(e) le recyclage d'au moins une partie du promoteur de catalyseur éliminé dans la distillation (b) à l'étape de mise en réaction (a).

7. Procédé selon l'une quelconque des revendications 1 à 6, la mise en contact (c) étant conduite à une température de 80 à 120 °C

8. Procédé selon l'une quelconque des revendications 1 à 7, la mise en contact (c) étant conduite par écoulement descendant du flux résiduel à travers un lit de résine d'échange d'anions basique.

9. Procédé selon l'une quelconque des revendications 1 à 8, la résine d'échange d'anions étant une résine d'échange anionique faiblement basique, contenant des groupes amine primaire, secondaire ou tertiaire.

10. Procédé selon l'une quelconque des revendications 1 à 9, la récupération (d) comprenant une cristallisation pour séparer le flux purifié en un produit solide contenant du bisphénol A et un flux de liqueur mère.

11. Procédé selon la revendication 10, le flux de liqueur mère comprenant d'autres isomères de diphénylolpropane que le bisphénol A et le procédé comprenant en outre :
(f) la mise en contact de la liqueur mère avec un catalyseur d'échange d'anions acide sulfoné dans les conditions pour convertir au moins une partie des autres isomères de diphénylolpropane en bisphénol A et pour produire un flux de produits isomérisés.

12. Procédé selon la revendication 11 et comprenant en outre :
(g) l'alimentation d'au moins une partie du flux de produits isomérisés à l'étape de mise en réaction (a).
